# EUROPEAN PATENT SPECIFICATION

(11) **EP 2 502 562 B1**
(45) Date of publication and mention of the grant of the patent: **02.09.2015**
(21) Application number: 12159565.6
(22) Date of filing: 15.03.2012
(51) Int. Cl.: A61B 6/00, A61B 6/10

(54) **Mobile X-ray apparatus provided with an anticollision device**
Ein mobiles Röntgengerät mit Anti-Kollisionsmittel
Appareil à rayons X mobile pourvu d'un dispositif d'anticollision

(30) Priority: 24.03.2011 FR 1152438
(43) Date of publication of application: 26.09.2012
(73) Proprietor: General Electric Company, Schenectady, NY 12345 (US)
(72) Inventor: Soto, José Emilio, 78530 Buc (FR); Guerit, Francis, 78530 Buc (FR); Bertrand, Guy, 78530 Buc (FR); Galloni, Bruno, 78530 Buc (FR); Bouvier, Bernard, 78530 Buc (FR)
(74) Representative: Bedford, Grant Richard

(56) References cited:
- EP-A1- 1 479 343
- US-A1- 2008 013 692
- US-A1- 2008 123 811
- US-A1- 2008 304 626
- US-B1- 7 046 764

## Description

The invention relates, in general, to X-ray apparatuses and, notably, to X-ray apparatuses used in the field of medical imaging.

It relates more particularly to X-ray apparatuses that are mounted on a mobile device.

X-ray apparatus conventionally comprise an X-ray tube and an X-ray detector placed opposite the X-ray tube in a direction of emission of the X-rays. The tube and the detector are usually placed on two mutually opposite ends of an arm.

Such apparatuses are used for angiographic examinations with a diagnostic or interventional aim.

During these examinations, it is necessary to produce radiographs of an area of interest in the body of a patient by X-rays. For this purpose, after the patient has been laid out on an examination table, the X-ray tube and the detector are brought to face the area to be radiographed.

In the prior art, there are several types of X-ray apparatuses making it possible to produce radiographs.

First, X-ray apparatuses are known that are fixed to the ground and in which the arm supporting the X-ray tube and the detector comprise several degrees of freedom making it possible to position the X-ray beam facing the area of interest.

This type of apparatus however has a major drawback relating to the fact that the radiography requirement is necessary only at the beginning and at the end of intervention. Meanwhile, it is access to the patient that should take precedence. They cannot therefore be removed from the examination table when they are not used. In particular, the transfer and installation of the patient on the examination table are hampered by the presence of this cumbersome system.

Moreover there are X-ray apparatuses called "surgical mobile" units that can be moved manually. In this case, they are mounted on a carriage that contains a certain number of batteries used to supply the X-ray tube with power. This type of apparatus is then not suitable for angiographic examinations because the necessary power delivered by the X-ray tube is no longer sufficient to obtain adequate image quality and, in particular, contrast.

Moreover, this type of mobile X-ray apparatus does not allow complex angulations because the diameter of the arm supporting the tube and the detector is not large enough. Similarly, these mobile X-ray apparatuses do not achieve sufficient rotation speeds to allow good-quality, three-dimensional image reconstructions. Finally, even though the weight of such an apparatus is half as much as that of an X-ray apparatus designed for angiography, it remains very difficult to move because of its relatively large dimensions and its weight, which can be up to 300 kg.

Moreover, X-ray apparatuses for angiography are known that are suspended from the ceiling and can be moved on guide rails, via a mobile carriage, for example with the aid of an electric motor. This type of apparatus also has several drawbacks.

First, many systems are already attached to the ceiling around the examination table, thus already cluttering the space around the patient, and making it difficult to install guide rails.

Secondly, mounting an X-ray apparatus on the ceiling considerably increases the risk of opportunistic contamination of the patient. Specifically, because of the difficulty of cleaning the rails, particles are likely to fall and contaminate the patient when the apparatus is sliding in the rails.

Moreover, in certain operating rooms, a sterile laminar flow is generated above the patient. In this case, the flow is likely to blow particles present on the rail which can then enter the laminar flow and reach the patient.

To alleviate these various drawbacks, it has been proposed to mount the X-ray apparatus on a mobile device mounted on wheels driven by drive motors controlled automatically under the control of a navigation system. In this respect it is possible to refer to document FR 2 945 724.

It has been found that such a system is particularly effective for moving the X-ray apparatus in an operating or examination room, notably to position the X-ray tube and the detector around the area of interest and to move it away when it is no longer in use, in order to free up the space around the examination table.

However, this X-ray apparatus, which is capable of being moved automatically, either in a stand-alone manner, or under the control of a control console that can be operated by an operator, in particular during its return to the out-of-the-way waiting position, is likely to come into contact with elements present on the floor of the operating room, such as control pedals, or to bump into the feet of the persons present in the operating room, which, because of the weight of the apparatus, which may be up to several hundreds of kilos, is likely either to damage the equipment of the operating room, or to cause injuries to the members of medical staff.

US 2008/0013692 describes an X-ray system with an industrial robot.

US 2008/0123811 describes a system and method for improved collision detection in an imaging device.

In the light of the foregoing, an object of various aspects of the invention is to propose an X-ray apparatus which remedies this drawback and which removes all risk of an accident likely to be caused by its automatic movement in the operating room.

The subject of certain aspects of the invention is therefore an X-ray apparatus as defined by the appended claims comprising an X-ray tube, an X-ray detector placed opposite the X-ray tube in a direction of emission of X-rays, and a mobile device on which the X-ray tube and the X-ray detector are mounted, the said device comprising driving means capable of causing the automatic movement of the X-ray apparatus.

According to a general feature of various aspects of the invention, the mobile device comprises an impact-sensing system, coupled to the driving means in order to control the movement of the mobile device in the event of impact applied to the X-ray apparatus.

By using an impact-sensing device, it is therefore possible, after having detected a collision, to control the mobile device, for example by stopping it or making it go into reverse in order to prevent any risk of injury or of damage to the equipment present in the operating room.

The impact-sensing system comprises a peripheral sensor mounted on a housing of the mobile device.

In one embodiment, the peripheral sensor comprises a resistive sensor.

The impact-sensing system may be mounted on the housing by means of a set of at least one elastically deformable element.

For example, the impact-sensing system may be mounted on the housing by means of springs.

The impact-sensing device may also comprise means for detecting the direction of the force of impact applied to the X-ray apparatus.

In one embodiment, the detection means may be means of detection by triangulation.

When the impact sensor is mobile relative to the housing of the mobile device, it is possible to make these detection means in the form of a set of elements for measuring the movement of the impact-sensing system relative to three fixed points linked to the mobile device, associated with computing means for determining the direction of the force of impact as a function of the said movement of the impact-sensing system.

The measuring elements may comprise a set of linear potentiometers.

Various objects, features and advantages of the invention will appear on reading the following description, given only as a non-limiting example and made with reference to the appended drawings in which:
Figure 1 is a schematic view of an X-ray apparatus provided with a mobile device fitted with an impact-sensing system according to the invention;
Figure 2 is a schematic view illustrating the mounting of the impact sensor onto the housing of the mobile device; and
Figure 3 shows the operating principle of the means of detection by triangulation.

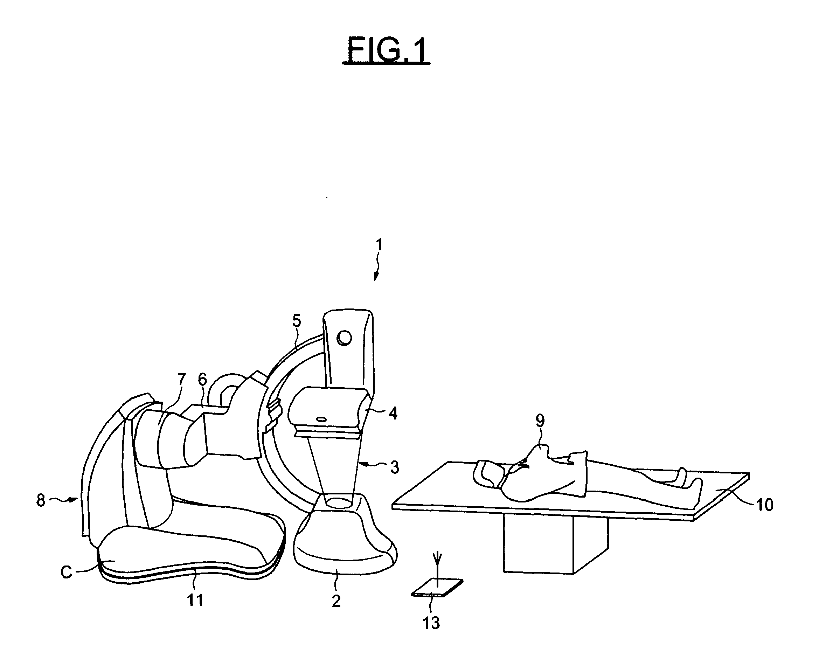

Figure 1 illustrates an X-ray apparatus 1 of the vascular type.

As can be seen, this apparatus 1 essentially comprises an X-ray tube 2, capable of emitting a beam 3 of X-rays in an emission direction, and a detector 4 of X-rays, placed at the two mutually opposite ends of an arm 5, in this instance in the shape of an arch, so that the X-rays emitted by the tube 2 are incident on the detector 4.

As can be seen, the arm 5 is mounted slidingly on a second arm 6 mounted so as to rotate on a fixed support 7, itself mounted on a mobile device 8.

Therefore, the support 7, the rotary arm 6 and the arm 5 are all three articulated relative to one another so that the X-ray apparatus can be moved in three dimensions and thus produce images of an organ to be examined at various angles of incidence.

During a radiograph, the tube 2 and the detector 4 are brought to face an area of interest of the body 9 of a patient laid out on an examination table 10 so that, when the area of interest is interposed between the X-ray tube 2 and the detector 4, it is irradiated by the X-rays, and the detector 4 produces representative data of features of the interposed area of interest.

The mobile device 8 comprises, in the exemplary embodiment shown, a housing C supported by a running system comprising, for example, two lateral drive and directional wheels placed at the rear, two free front wheels (not visible in Figure 1), and means for driving the drive wheels placed in the housing C and comprising a directional motor coupled to a drive motor. The mobile device 8 is a programmable device and is associated with a navigation system capable, for example, of communicating by radioelectric link with identification devices 13 placed in the operating room in order to allow the apparatus 1 to be located precisely in the room and, notably, relative to the examination table 10.

Therefore, according to the programming phases or under the control of a control console that can be operated by an operator, the X-ray apparatus is capable of being moved automatically in the operating room.

This is particularly the case, notably, during the positioning of the X-ray apparatus facing the examination table in order to place the tube 2 and the detector 4 facing an area of interest to be radiographed or during the movement of the X-ray apparatus to an out-of-the-way waiting position when it is no longer in use.

For the purpose of avoiding any accident in the case of a collision either with equipment, of the control pedal type, present on the floor of the operating room or to avoid any injury in the case of bumping into a person present in the room, the X-ray apparatus is provided with an impact-sensing system coupled to the means for driving the drive wheels of the mobile device.

This impact-sensing system is provided on the sensor C of the mobile device so as to detect the impacts likely to occur during the movement of the apparatus 1 and cause it to stop, or even to reverse, if an impact is detected.

As can be seen in Figure 1, the impact-sensing system comprises a peripheral sensor 11 mounted in the bottom portion of the housing C, protruding laterally from the latter.

In one embodiment, this peripheral sensor is made in the form of a resistive sensor comprising, for example, a pair of conductive tracks place in parallel in a rubber sleeve and capable of coming into contact in the event of impact.

For example, an impact can then be detected by powering one of the tracks, and by detecting the presence of a current in the other track in the event of the two conductive tracks being placed in localized contact.

Naturally, other embodiments of the impact sensor can also be envisaged. Therefore, there is no departure from the context of the invention when other types of sensors, for example optical, inductive, etc. sensors are used that are capable of delivering an impact-detection signal.

The detection signal thus generated can then be transmitted to a control circuit for controlling the means for driving the wheels of the mobile device so as to stop the X-ray apparatus or drive it in reverse.

It will be noted that the embodiment of the impact sensor in the form of a sensor that is for example resistive, provided over the whole periphery of the housing, makes it possible to detect impacts on 360°, irrespective of the point of impact.

However, there is no further departure from the context of the invention when the peripheral impact sensor is made in the form of a set of individual sensors distributed over the whole periphery of the housing.

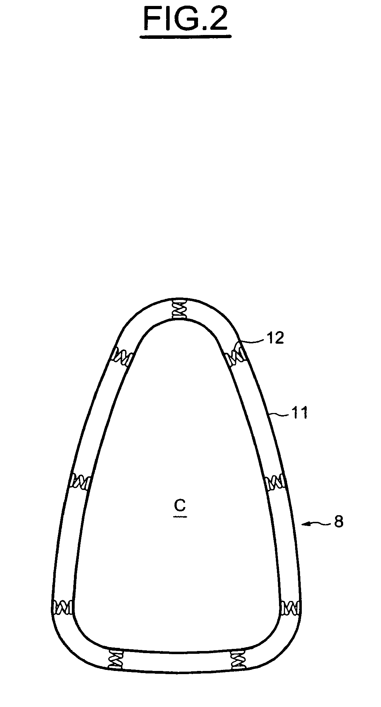

With reference to Figure 2, in one advantageous embodiment, the peripheral sensor 11 is mounted on the housing C by means of elastically deformable mounting elements, such as 12, in this instance springs evenly distributed between the peripheral sensor 11 and the housing C along its periphery.

These elastically deformable elements have the effect, on the one hand, of absorbing the impacts and, on the other hand, of compensating for the necessary delay in the complete stopping of the apparatus in order to prevent, because of this time delay, the housing from reaching the element that has been collided with.

As indicated above, it may be advisable, in the event of impact, to cause the mobile device 8 to reverse in order to clear away the element collided with.

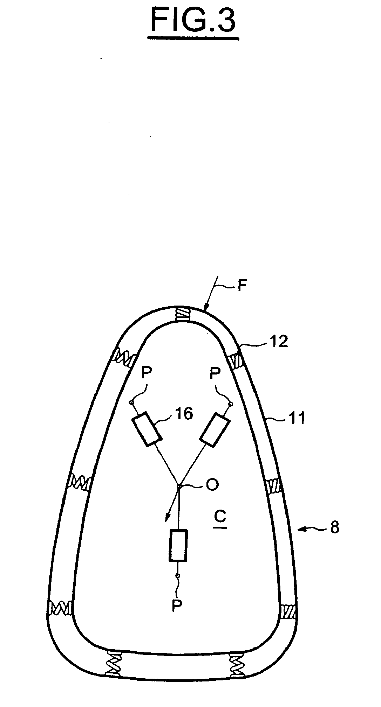

For this purpose, the anticollision system is also provided with detection means capable of detecting the direction of the force (arrow F) applied to the impact sensor 11 (Figure 3).

Various detection means can be used for this purpose.

Advantageously use will be made of means of detection by triangulation.

It will be possible, for example, to use a set of linear potentiometers, such as 16, in this instance three in number, mounted on the one hand on a fixed point, such as P, linked to the housing C of the mobile device 8 and, on the other hand, to a common point O linked to a support of the impact sensor 11.

Therefore, in the event of impact, a measurement circuit detects the resulting change in resistance of each of the linear potentiometers 16, and converts this measurement into a corresponding distance measurement between each of the fixed points P and the central point O which is coupled to the impact sensor 11. These distance measurements make it possible to obtain, by direct triangulation, the change in position of the central point O and therefore the direction of application of the force. In this way the directional wheels and the driving means of the drive wheels of the mobile device 8 are directly controlled in order to cause a reverse movement and thus release the element collided with.

As can be understood, the embodiments of the invention that have just been described, which comprise an X-ray tube, an X-ray detector placed opposite the X-ray tube in a direction of emission of X-rays, and a mobile device on which the X-ray tube and the X-ray detector are mounted, in which the said mobile device comprises at least one motor capable of causing the automatic movement of the X-ray apparatus, and in which the mobile device comprises an impact-sensing system coupled to the motor in order to control the movement of the mobile device in the event of impact applied to the X-ray apparatus, makes it possible to detect impacts that might occur when the mobile device 8 moves.

When the impact-sensing system is provided with detection means capable of detecting the direction of the applied force, it is also possible to achieve a reverse movement.

But it will be noted that, in the embodiment in which a system of triangulation based on the use of potentiometers is used, the peripheral-impact sensor may be omitted because the means for detecting the direction of the impact also make it possible to detect the application of a force.

It will be noted finally that the invention is not limited to the embodiment described.

The means of detection by triangulation, based on the use of linear potentiometers, may be replaced by any other type of triangulation device, for example based on the use of pulse encoders.

## Claims

1. An X-ray apparatus (1), comprising an X-ray tube (2), an X-ray detector (4) placed opposite the X-ray tube (2) in a direction of emission of X-rays, and a mobile device (8) on which the X-ray tube and the X-ray detector are mounted, the said mobile device comprising driving means capable of causing the automatic movement of the X-ray apparatus, wherein the mobile device comprises an impact-sensing system coupled to the driving means in order to control the movement of the mobile device in the event of impact applied to the X-ray apparatus, **characterised in that** the impact-sensing system comprises a peripheral sensor (11) mounted on a housing (C) of the mobile device which extends over the whole periphery of the housing (C).

2. The X-ray apparatus (1) according to Claim 1, wherein the peripheral sensor (11) comprises a resistive sensor.

3. The X-ray apparatus according to Claim 1 or Claim 2, wherein the impact-sensing system is mounted on the housing (C) of the mobile device by means of a set of elastically deformable elements (12).

4. The X-ray apparatus (1) according to Claim 3, wherein the impact-sensing system (11) is mounted on the housing (C) by means of springs.

5. The X-ray apparatus (1) according to any one of Claims 1 to 4, further comprising means for detecting the direction of the force of impact applied to the X-ray apparatus.

6. The X-ray apparatus (1) according to Claim 5, wherein said detection means are means of detection by triangulation.

7. The X-ray apparatus (1) according to Claim 6, wherein the means of detection by triangulation comprise a set of elements (16) for measuring the movement of the impact-sensing system relative to three fixed points linked to the mobile device and computing means for determining the direction of the force of impact as a function of the said movement of the impact-sensing system.

8. The X-ray apparatus (1) according to Claim 7, wherein the measuring elements (16) comprise a set of linear potentiometers.

## Patentansprüche

1. Röntgengerät (1), umfassend eine Röntgenröhre (2), einen gegenüber der Röntgenröhre (2) in Richtung der Ausstrahlung von Röntgenstrahlen angeordneten Röntgenstrahlendetektor (4), und eine Mobilvorrichtung (8), an der die Röntgenröhre und der Röntgenstrahlendetektor gelagert sind, wobei die Mobilvorrichtung Antriebsmittel umfasst, die in der Lage sind, die automatische Bewegung des Röntgengeräts zu bewirken, wobei die Mobilvorrichtung ein Stoßerfassungssystem aufweist, das mit den Antriebsmitteln gekoppelt ist, um die Bewegung der Mobilvorrichtung in dem Fall zu steuern, in dem ein Stoß auf das Röntgengerät aufgebracht wird, **dadurch gekennzeichnet, dass** das Stoßerfassungssystem einen auf einem Gehäuse (C) der Mobilvorrichtung gelagerten peripheren Sensor (11) aufweist, der sich über die gesamte Peripherie des Gehäuses (C) erstreckt.

2. Röntgengerät (1) nach Anspruch 1, wobei der periphere Sensor (11) einen resistiven Sensor umfasst.

3. Röntgengerät nach Anspruch 1 oder 2, wobei das Stoßerfassungssystem unter Einsatz eines Satzes von elastisch verformbaren Elementen (12) auf dem Gehäuse (C) der mobilen Vorrichtung gelagert ist.

4. Röntgengerät (1) nach Anspruch 3, wobei das Stoßerfassungssystem (11) unter Einsatz von Federn auf dem Gehäuse (C) gelagert ist.

5. Röntgengerät (1) nach einem der Ansprüche 1 bis 4, ferner umfassend Mittel zum Erfassen der Richtung der Kraft des Stoßes, der auf das Röntgengerät aufgebracht wird.

6. Röntgengerät (1) nach Anspruch 5, wobei die Erfassungsmittel Mittel zur Erfassung durch Triangulierung sind.

7. Röntgengerät (1) nach Anspruch 6, wobei die Mittel zum Erfassen durch Triangulierung einen Satz Elemente (16) zum Messen der Bewegung des Stoßerfassungssystems mit Bezug auf drei mit der Mobilvorrichtung verbundene feste Punkte und Berechnungsmittel zum Bestimmen der Richtung der Kraft des Stoßes als Funktion der Bewegung des Stoßerfassungssystems aufweisen.

8. Röntgengerät (1) nach Anspruch 7, wobei die Messelemente (16) einen Satz lineare Potenziometer umfassen.

## Revendications

1. Appareil à rayons X (1), comprenant un tube à rayons X (2), un détecteur de rayons X (4) placé à l'opposé du tube à rayons X (2) dans le sens d'émission des rayons X, et un dispositif mobile (8) sur lequel le tube à rayons X et le détecteur de rayons X sont montés, ledit dispositif mobile comprenant des moyens d'entraînement qui sont à même de provoquer le mouvement automatique de l'appareil à rayons X, dans lequel le dispositif mobile comprend un système de détection de collisions couplé aux moyens d'entraînement afin de commander le mouvement du dispositif mobile dans le cas d'une collision appliquée à l'appareil à rayons X, **caractérisé en ce que** le système de détection de collisions comprend un capteur périphérique (11) monté sur un boîtier (C) du dispositif mobile qui s'étend sur toute la périphérie du boîtier (C).

2. Appareil à rayons X (1) selon la revendication 1, dans lequel le capteur périphérique (11) comprend un capteur résistif.

3. Appareil à rayons X (1) selon la revendication 1 ou la revendication 2, dans lequel le système de détection de collisions est monté sur le boîtier (C) du dispositif mobile au moyen d'un ensemble d'éléments à déformation élastique (12).

4. Appareil à rayons X (1) selon la revendication 3, dans lequel le système de détection de collisions (11) est monté sur le boîtier (C) au moyen de ressorts.

5. Appareil à rayons X (1) selon l'une quelconque des revendications 1 à 4, comprenant en outre des moyens pour détecter la direction de la force de la collision appliquée à l'appareil à rayons X.

6. Appareil à rayons X (1) selon la revendication 5, dans lequel lesdits moyens de détection sont des moyens de détection par triangulation.

7. Appareil à rayons X (1) selon la revendication 6, dans lequel les moyens de détection par triangulation comprennent un ensemble d'éléments (16) pour mesurer le mouvement du système de détection de collisions relatif à trois points fixes liés au dispositif mobile et des moyens de calcul pour déterminer la direction de la force de collision en fonction dudit mouvement du système de détection de collisions.

8. Appareil à rayons X (1) selon la revendication 7, dans lequel les éléments de mesure (16) comprennent un ensemble de potentiomètres linéaires.
